# EUROPEAN PATENT APPLICATION

(11) **EP 2 949 260 A1**
(43) Date of publication of application: **02.12.2015**
(21) Application number: 14847164.2
(22) Date of filing: 23.04.2014
(51) Int. Cl.: A61B 1/00, A61B 1/06

(54) **ENDOSCOPE AND ENDOSCOPIC SYSTEM**

(30) Priority: 27.09.2013 JP 2013201899
(71) Applicant: OLYMPUS CORPORATION, Shibuya-ku Tokyo 151-0072 (JP)
(72) Inventor: NAKAJIMA Sho, Tokyo 151-0072 (JP)
(74) Representative: Schicker, Silvia
(86) International application number: PCT/JP2014/061423
(87) International publication number: WO 2015/045463

(57) **Abstract**

An endoscope 1 includes: an insertion portion 2; a distal end face 14a that can receive light from an observed body; a detection fiber 14 that transmits light received by the distal end face 14a; an opening 17 that opens near the distal end face 14a; a treatment instrument insertion channel 16 inserted into the insertion portion 2; an endoscope main body portion 3 consecutively connected with the insertion portion 2; a treatment instrument insertion port 7 that is provided on the endoscope main body portion 3 and that includes an opening communicating with the treatment instrument insertion channel 16; an external detection fiber 10 that is provided on the endoscope main body portion 3 and that can guide light between outside and inside of the endoscope main body portion 3; and a merging portion 15 that is provided inside of the endoscope main body portion 3 and that merges the light transmitted by the detection fiber 14 and the light guided by the external detection fiber 10.

## Description

### Technical Field

The present invention relates to an endoscope and an endoscope system for scanning with laser light.

### Background Art

As is well known, there is an electronic endoscope, in which an image pickup apparatus including a solid image pickup device, such as a CCD and a CMOS, photoelectrically converts a subject image and displays an acquired image on a monitor. In recent years, an example of an apparatus that displays an object image without using a technique of the solid image pickup device includes a scanning endoscope. The scanning endoscope causes a distal end of an illumination fiber that guides illuminating light from a light source to scan. A detection fiber arranged around the illumination fiber receives return light from a subject, and a light intensity signal detected over time is used to form an object image.

In the scanning endoscope, a drive section, such as an actuator, that displaces the illumination fiber for scanning with illuminating light is provided in a distal end portion of an insertion portion, and the illuminating light, such as laser light, is applied from an illumination lens at a distal end of the insertion portion to illuminate an object.

For example, Japanese Patent Application Laid-Open Publication No. 2011-115252 discloses a scanning medical probe including a single-mode fiber that transfers illuminating light into a body cavity, wherein an object is scanned by a predetermined scan pattern by emitting the illuminating light in a state in which a distal end of the single-mode fiber is resonated.

However, with only the number of detection fibers that can be mounted, brightness may be insufficient in the scanning medical probe due to limitations in diameter. Although the brightness can be secured when a relatively near point is observed after arrival to a target site, the brightness is particularly insufficient when a relatively far point is observed before arrival to the target site.

If the number of detection fibers is increased to secure sufficient brightness, the outer diameter becomes thick, and insertion to a thin conduit becomes impossible. Further, if an amount of laser light for illumination is increased to secure sufficient brightness, a laser light amount more than a stipulated amount may be applied to the object.

Thus, an object of the present invention is to provide an endoscope and an endoscope system that can prevent thickening of an outer diameter of an insertion portion and that can secure brightness necessary for reaching a target site without application of laser light at an unnecessarily high level.

### Disclosure of Invention

### Means for Solving the Problem

An aspect of the present invention provides an endoscope including: a long insertion portion that is inserted into an observed section and that includes a distal end portion and a proximal end portion; a first light receiving portion that is provided on the distal end portion and that can receive light from an observed body; a first light transmission portion that is inserted into the insertion portion and that transmits light received by the first light receiving portion; a distal end side opening that opens near the first light receiving portion; a conduit that communicates with the distal end side opening and that is inserted into the insertion portion; a proximal end side main body portion provided on a proximal end side relative to the proximal end portion and consecutively connected with the insertion portion; a proximal end side opening that is provided on the proximal end side main body portion and that includes an opening communicating with the conduit; a light guiding portion that is provided on the proximal end side main body portion and that can guide light between outside and inside of the proximal end side main body portion; and a merging portion that is provided inside of the proximal end side main body portion and that merges the light transmitted by the first light transmission portion and the light guided by the light guiding portion.

Further, an aspect of the present invention provides an endoscope system including: an endoscope including: a long insertion portion that is inserted into an observed section and that includes a distal end portion and a proximal end portion; a first light receiving portion that is provided on the distal end portion and that can receive light from an observed body; a first light transmission portion that is inserted into the insertion portion and that transmits light received by the first light receiving portion; a distal end side opening that opens near the first light receiving portion; a conduit that communicates with the distal end side opening and that is inserted into the insertion portion; a proximal end side main body portion provided on a proximal end side relative to the proximal end portion and consecutively connected with the insertion portion; a proximal end side opening that is provided on the proximal end side main body portion and that includes an opening communicating with the conduit; and a light guiding portion that is provided on the proximal end side main body portion and that can guide light between outside and inside of the proximal end side main body portion; and a main body apparatus including a merging portion that merges the light transmitted by the first light transmission portion and the light guided by the light guiding portion.

### Brief Description of the Drawings

Fig. 1 is a diagram for describing an overall configuration of an endoscope according to a first embodiment;
Fig. 2 is a diagram for describing a distal end configuration of the endoscope;
Fig. 3 is a cross-sectional view for describing a connection configuration of an external detection fiber and a connector;
Fig. 4 is a diagram for describing an electrical configuration of an endoscope system including an endoscope 1;
Fig. 5 is a diagram for describing a configuration of an endoscope system according to a modification of the first embodiment;
Fig. 6 is a diagram showing a configuration of an endoscope according to a second embodiment;
Fig. 7 is a cross-sectional view for describing a connection configuration of an external detection fiber connection portion and a connection portion of a connector;
Fig. 8A is a diagram for describing another connection configuration of the external detection fiber connection portion and the connection portion of the connector;
Fig. 8B is a diagram for describing the other connection configuration of the external detection fiber connection portion and the connection portion of the connector;
Fig. 9 is a diagram for describing a configuration of the connection portion of the connector;
Fig. 10A is a diagram for describing another configuration of the connection portion of the connector; and
Fig. 10B is a diagram for describing the other configuration of the connection portion of the connector.

### Best Mode for Carrying Out the Invention

Hereinafter, embodiments of the present invention will be described with reference to the drawings.

### (First Embodiment)

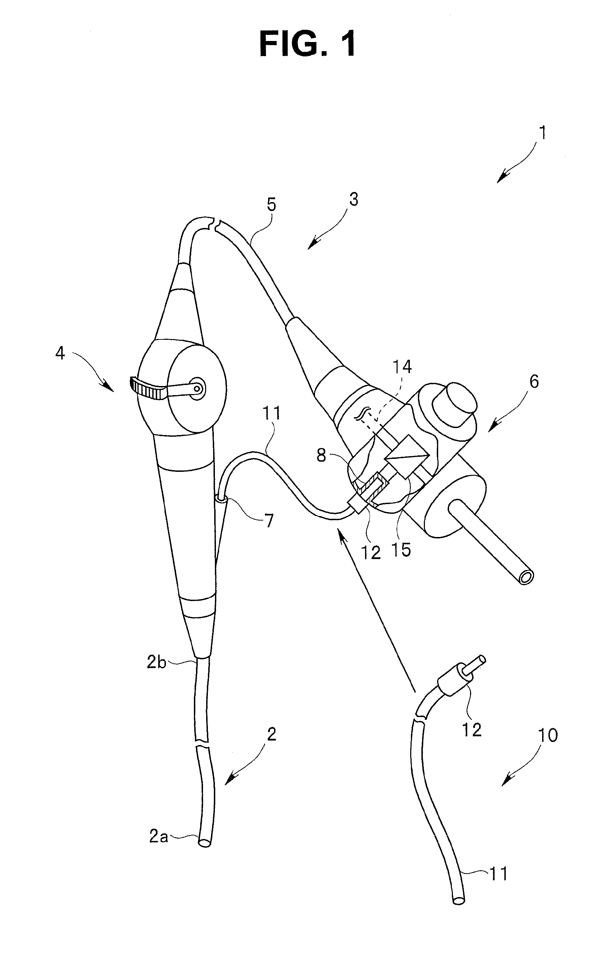

First, an overall configuration of an endoscope of a first embodiment will be described with reference to Figs. 1 to 3.

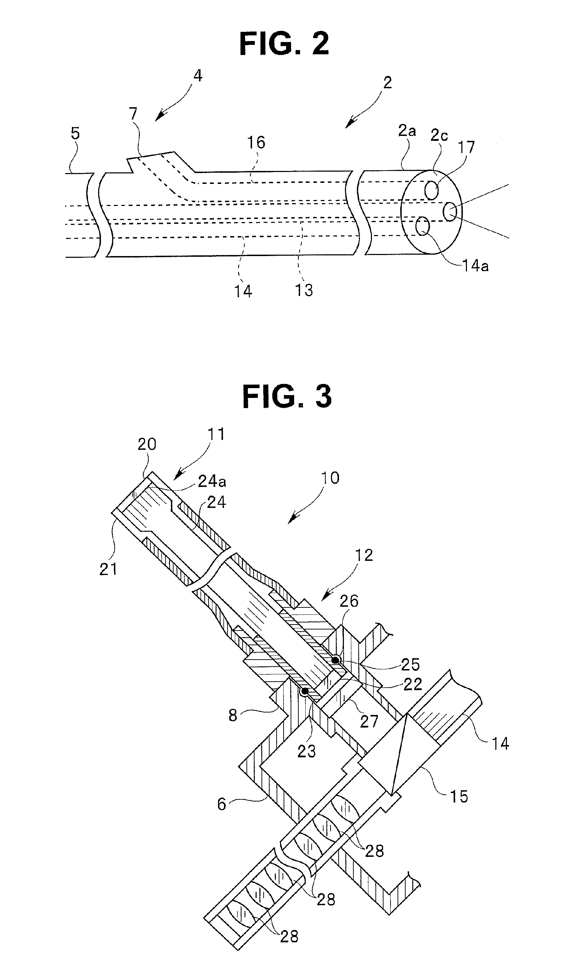

Fig. 1 is a diagram for describing the overall configuration of the endoscope according to the first embodiment. Fig. 2 is a diagram for describing a distal end configuration of the endoscope. Fig. 3 is a cross-sectional view for describing a connection configuration of an external detection fiber and a connector.

An endoscope 1 shown in Fig. 1 is a scanning endoscope that applies laser light (illuminating light) to an observed body while performing scanning with the laser light to obtain return light from the observed body. The endoscope 1 includes: a long insertion portion 2 that is inserted into an observed section and that includes a distal end portion 2a and a proximal end portion 2b; and an endoscope main body portion 3 provided on a proximal end side relative to a proximal end portion and consecutively connected with the insertion portion 2.

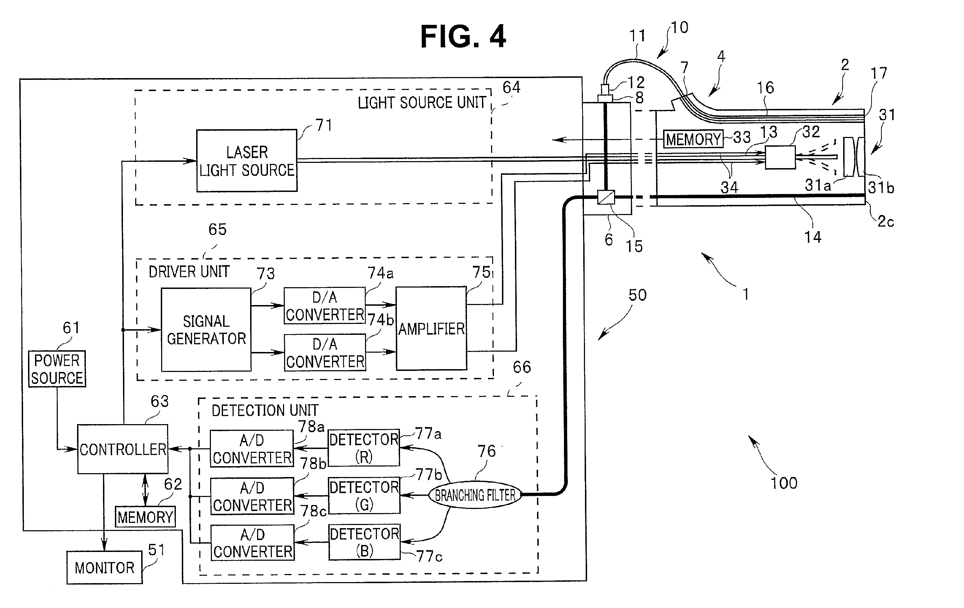

The endoscope main body portion 3 as a proximal end side main body portion includes: an operation portion 4 consecutively connected with the proximal end portion 2b of the insertion portion 2; a long flexible tube portion 5 consecutively connected with a proximal end side of the operation portion 4; and a connector 6 consecutively connected with a proximal end side of the flexible tube portion 5. The connector 6 can be attached to and detached from a main body apparatus 50 (see Fig. 4) described later.

The operation portion 4 is provided with a treatment instrument insertion port 7 for inserting a treatment instrument, such as forceps, into the insertion portion 2. The treatment instrument insertion port 7 serves as a proximal end side opening including an opening communicating with a treatment instrument insertion channel 16 (see Fig. 2) described later.

Further, the connector 6 is provided with a connection portion 8 (first connection portion) to which a connection portion 12 of an external detection fiber 10 described later can be attached and detached. The external detection fiber 10 includes: an insertion portion 11; and the connection portion 12 (second connection portion) that is provided on a proximal end side of the insertion portion 11 and that can be attached to and detached from the connection portion 8 of the connector 6.

As shown in Fig. 2, an optical fiber 13 that guides light from a laser light source 71 (see Fig. 4) of the main body apparatus 50 described later to apply laser light to an observed body is inserted into the insertion portion 2. Further, a detection fiber 14 that receives the return light from the observed body through a distal end face 14a as a first light receiving portion is inserted into the insertion portion 2. The detection fiber 14 is also inserted into the endoscope main body portion 3, and a proximal end portion communicates with a merging portion 15 in the connector 6. The detection fiber 14 as a first light transmission portion transmits (guides) the return light received by the distal end face 14a to the merging portion 15.

Further, the treatment instrument insertion channel 16 as a conduit that communicates with the treatment instrument insertion port 7 and that allows insertion of a treatment instrument is inserted into the insertion portion 2. Furthermore, an opening 17 as a distal end side opening including an opening communicating with the treatment instrument insertion channel 16 is provided near the distal end face 14a of the detection fiber 14.

The insertion portion 11 of the external detection fiber 10 is inserted into the treatment instrument insertion channel 16 from the treatment instrument insertion port 7 and the connection portion 12 is connected to the connection portion 8 of the connector 6 so that the return light from the observed body can be received and guided to the merging portion 15 provided in the connector 6. The external detection fiber 10 serves as a light guiding portion that can guide the return light from the observed body between outside and inside of the endoscope main body portion 3.

As shown in Fig. 3, in the external detection fiber 10, a lens frame 21 fixes a lens 20 to a distal end portion of the insertion portion 11. Further, in the external detection fiber 10, a lens frame 23 fixes a lens 22 also serving as a cover glass to a proximal end portion of the connection portion 12. Then, a detection fiber 24 as a second light transmission portion that guides the return light from the observed body is provided between the lens 20 and the lens 23. The detection fiber 24 receives the return light from the observed body through a distal end face 24a as a second light receiving portion consecutively connected with the lens 20.

A locking ring 25 is provided on a proximal end side of the connection portion 12. Further, a locking groove 26 fitted with the locking ring 25 is provided on an inner wall of the connection portion 8 of the connector 6. In this way, the locking ring 25 is fitted with the locking groove 26 when the connection portion 12 of the external detection fiber 10 is connected to the connection portion 8 of the connector 6.

A cover glass 27 is provided at a position opposing the lens 22 when the connection portion 12 of the external detection fiber 10 is connected to the connection portion 8 of the connector 6. The return light from the observed body guided by the detection fiber 24 of the external detection fiber 10 is guided to the merging portion 15 through the lens 22 and the cover glass 27.

Further, a proximal end portion of the detection fiber 14 is consecutively connected with the merging portion 15, and the return light from the observed body is guided from the detection fiber 14. In this way, the return light from the detection fiber 14 is guided to the merging portion 15 provided in the connector 6. The insertion portion 11 of the external detection fiber 10 is inserted into the treatment instrument insertion channel 16, and the return light from the external detection fiber 10 is also guided to the merging portion 15.

The merging portion 15 is, for example, a splitter that merges the return light from the detection fiber 14 and the return light from the external detection fiber 10. The return light merged by the merging portion 15 is guided to the main body apparatus 50 described later through a relay lens group 28.

Note that the positions for arranging the connection portion 8 and the merging portion 15 are not limited to the connector 6, and it is only necessary that the connection portion 8 and the merging portion 15 are provided between the operation portion 4 and the connector 6. That is, it is only necessary that the return light from the detection fiber 14 and the return light from the external detection fiber 10 are merged in the endoscope main body portion 3.

In particular, the connection portion 8 and the merging portion 15 can be provided near the operation portion 4 or the treatment instrument insertion port 7 provided on the operation portion 4. With such a configuration, when the insertion portion 2 reaches a target site, the insertion portion 11 of the external detection fiber 10 can be immediately pulled out from the treatment instrument insertion channel 16 and can be replaced with the treatment instrument. This can improve operability.

Here, an electrical configuration of an endoscope system including the endoscope 1 will be described. Fig. 4 is a diagram for describing the electrical configuration of the endoscope system including the endoscope 1.

As shown in Fig. 4, an endoscope system 100 includes: the endoscope 1; the main body apparatus 50 connected to the endoscope 1; and a monitor 51 that displays a subject image obtained by the main body apparatus 50. As described, the endoscope 1 can be attached to and detached from the main body apparatus 50 through the connector 6.

A distal end optical system 31 including illumination lenses 31a and 31 b is provided on a distal end face 2c of the insertion portion 2. The distal end optical system 31 includes the illumination lens 31a that is a plano-convex lens and the illumination lens 31b that is a convex-planar lens arranged in this order from a side of the laser light source 71 described later. Note that although the distal end optical system 31 includes two illumination lenses 31a and 31b, the arrangement is not limited to this, and the distal end optical system 31 may include one illumination lens or three or more illumination lenses.

Further, on a distal end side of the optical fiber 13 that guides the light from the laser light source 71 described later to apply laser light to the observed body, an actuator 32 that causes a distal end of the optical fiber 13 to scan in a desired direction based on a drive signal from a driver unit 65 described later is provided. Although not shown, for example, a ferrule in a shape of a square pole is arranged between the optical fiber 13 and the actuator 32, and the actuator 32 is arranged on each side of the ferrule.

The ferrule is a member used in a field of optical communication, and zirconia (ceramic), nickel, or the like is used as a material. Center hole processing with a high accuracy (for example, ±1 µm) with respect to an outer diameter (for example, 125 µm) of the optical fiber 13 can be easily realized. Then, a through hole based on the diameter of the optical fiber 13 is provided at an approximate center of the ferrule, and the optical fiber 13 is fixed by an adhesive or the like.

Further, a memory 33 storing various information related to the endoscope 1 is provided inside of the operation portion 4. The memory 33 is connected to a controller 63 described later through a signal line not shown when the endoscope 1 is connected to the main body apparatus 50, and the controller 63 reads various information related to the endoscope 1.

A plurality of conductor wires 34 formed by, for example, linear metal are deposited on the optical fiber 13, from the connector 6 to the actuator 32. The actuator 32 is provided at distal ends of the plurality of conductor wires 34. Then, the plurality of conductor wires 34 are connected to an amplifier 75 described later of the main body apparatus 50 when the connector 6 is fitted to the main body apparatus 50. The plurality of conductor wires 34 supply the actuator 32 with a drive signal for driving the actuator 32 outputted from the amplifier 75.

The main body apparatus 50 includes a power source 61, a memory 62, a controller 63, a light source unit 64, the driver unit 65, and a detection unit 66.

The light source unit 64 includes the laser light source 71. Further, the driver unit 65 includes a signal generator 73, digital/analog (hereinafter, called D/A) converters 74a and 74b, and the amplifier 75.

The detection unit 66 includes a demultiplexer 76, detectors 77a to 77c, and analog/digital (hereinafter, called A/D) converters 78a to 78c.

The power source 61 controls supply of power to the controller 63 according to operation of a power switch or the like not shown. A control program and the like for controlling the entire main body apparatus 50 is stored in the memory 62.

When the power is supplied from the power source 61, the controller 63 reads the control program from the memory 62 and controls the light source unit 64 and the driver unit 65. The controller 63 also analyzes light intensity of the return light from the object detected by the detection unit 66 and controls the monitor 51 to display an obtained object image.

The laser light source 71 of the light source unit 64 emits laser light (illuminating light) of a predetermined wavelength band to the optical fiber 13 based on the control by the controller 63. The optical fiber 13 emits the laser light (illuminating light) from the laser light source 71 to the observed body through the illumination lenses 31a and 31b.

Based on the control by the controller 63, the signal generator 73 of the driver unit 65 outputs a drive signal for causing the distal end of the optical fiber 13 to scan in a desired direction, or for example, to perform spiral scan (scan drive). More specifically, the signal generator 73 outputs, to the D/A converter 74a, a drive signal for causing the distal end of the optical fiber 13 to drive in a horizontal direction (X axis direction) with respective to an insertion axis of the insertion portion 2 and outputs, to the D/A converter 74b, a drive signal for causing the distal end of the optical fiber 13 to drive in a vertical direction (Y axis direction) with respect to the insertion axis of the insertion portion 2.

The D/A converters 74a and 74b convert the inputted drive signals from digital signals to analog signals and output the signals to the amplifier 75. The amplifier 75 amplifies the inputted drive signals and outputs the signals to the actuator 32. The drive signals outputted from the amplifier 75 are supplied to the actuator 32 through the plurality of conductor wires 34 deposited on the optical fiber 13.

Based on the drive signals from the amplifier 75, the actuator 32 causes the distal end of the optical fiber 13 to swing and perform spiral scan. More specifically, the actuator 32 vibrates with the optical fiber 13 and drives the optical fiber 13 in a radial direction so as to scan a site to be observed with the illuminating light emitted by the optical fiber 13. In this way, the light emitted to the optical fiber 13 from the laser light source 71 of the light source unit 64 is sequentially spirally applied to the observed body.

The detection fiber 14 receives the return light reflected by a surface region of the observed body and guides the received return light to the merging portion 15. Further, when the external detection fiber 10 is connected to the endoscope 1, the external detection fiber 10 receives the return light reflected by the surface region of the observed body and guides the received return light to the merging portion 15.

The merging portion 15 merges the return light guided from the detection fiber 14 and the return light guided from the external detection fiber 10 and guides the light to the demultiplexer 76. Note that when the external detection fiber 10 is not connected to the endoscope 1, the merging portion 15 guides the return light guided from the detection fiber 14 to the demultiplexer 76 as it is.

The demultiplexer 76 is, for example, a dichroic mirror that splits the return light by predetermined wavelength bands. More specifically, the demultiplexer 76 splits the return light guided from the merging portions 15 into return lights of R, G and B wavelength bands and outputs the return lights to the detectors 77a, 77b, and 77c, respectively.

The detectors 77a, 77b, and 77c detect light intensities of the return lights of the R, G and B wavelength bands, respectively. Signals of the light intensities detected by the detectors 77a, 77b, and 77c are outputted to the A/D converters 78a, 78b, and 78c, respectively.

The A/D converters 78a to 78c convert the signals of the light intensities outputted from the detectors 77a to 77c, respectively, from analog signals to digital signals and output the digital signals to the controller 63.

The controller 63 applies predetermined image processing to the digital signals from the A/D converters 78a to 78c to generate an object image and displays the object image on the monitor 51.

Here, effects of the endoscope 1 configured in this way will be described.

First, before inserting the insertion portion 2 into the observed body, an operator inserts the insertion portion 11 of the external detection fiber 10 into the treatment instrument insertion channel 16 and connects the connection portion 12 of the external detection fiber 10 to the connection portion 8 of the connector 6 to attach the external detection fiber 10 to the endoscope 1. Subsequently, the operator inserts the insertion portion 2 of the endoscope 1 into the target site of the observed body.

While the insertion portion 2 is being inserted into the target site, the observation is at a relatively far point with insufficient brightness. In this case, since the external detection fiber 10 is attached to the endoscope 1, the return light from the observed body received by the light receiving surface of the detection fiber 24 is guided to the merging portion 15. Further, the return light from the observed body received by the light receiving surface of the detection fiber 14 inserted into the insertion portion 2 is also guided to the merging portion 15.

The merging portion 15 merges the return light from the external detection fiber 10 and the return light from the detection fiber 14 guided in this way. The detection unit 66 of the main body apparatus 50 detects the light intensity of the return light merged by the merging portion 15, and the controller 63 applies predetermined image processing to the return light. Then, the return light is displayed on the monitor 51. In this way, when the external detection fiber 10 is attached to the endoscope 1, the merging portion 15 merges the return light from the external detection fiber 10 and the return light from the detection fiber 14, and necessary brightness is secured.

Next, when the insertion portion 2 reaches the target site, a relatively near point is observed, and sufficient brightness can be secured just by the return light from the detection fiber 14. Therefore, the operator removes the external detection fiber 10 from the endoscope 1 when the insertion portion 2 reaches the target site. That is, the operator pulls out the insertion portion 11 of the external detection fiber 10 from the treatment instrument insertion channel 16 and removes the connection portion 12 of the external detection fiber 10 from the connection portion 8 of the connector 6. Then, the operator inserts the treatment instrument into the treatment instrument insertion channel 16 as necessary to treat the lesion site or the like.

In this way, in the endoscope 1, the insertion portion 11 of the external detection fiber 10 is inserted into the treatment instrument insertion channel 16 to guide the return light from the observed body. Therefore, a detection fiber that guides the return light does not have to be newly provided in the insertion portion 2 of the endoscope 1, and thickening of the insertion portion 2 can be prevented.

Further, the endoscope 1 merges the return light from the external detection fiber 10 and the return light from the detection fiber 14 to secure the brightness. Therefore, an amount of the laser light does not have to be increased, and the brightness can be safely secured.

Therefore, according to the endoscope of the present embodiment, thickening of the outer diameter of the insertion portion can be prevented, and brightness necessary for reaching the target site can be secured without application of laser light at an unnecessarily high level.

Note that contrary to the present embodiment, an external illumination fiber can be inserted into the treatment instrument insertion channel to secure the brightness in a conventional endoscope using an image pickup device. However, with such a configuration, strong light for illumination is guided to the illumination fiber. Therefore, the illumination fiber may generate heat, and a burn or the like may occur. On the other hand, the external detection fiber 10 guides the return light in the present embodiment, and the brightness can be safely secured without heat generation by the external detection fiber 10.

### (Modification)

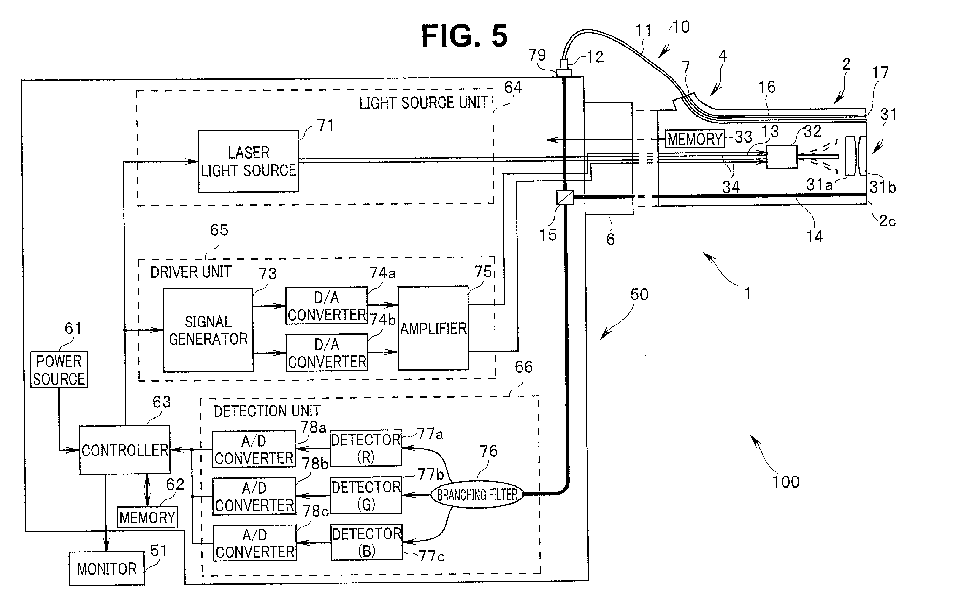

Fig. 5 is a diagram for describing a configuration of an endoscope system according to a modification of the first embodiment. Note that the same components as in Fig. 4 are designated with the same reference numerals in Fig. 5, and the description will be omitted.

An endoscope system 100a shown in Fig. 5 includes an endoscope 1 a and a main body apparatus 50a connected to the endoscope 1a.

From the endoscope 1 to the connection portion 8 and the merging portion 15 of Fig. 4 are deleted in the endoscope 1a. Further, the main body apparatus 50a is formed by adding, to the main body apparatus 50 of Fig. 4: a connection portion 79 to which the connection portion 12 of the external detection fiber 10 can be attached and detached; and the merging portion 15 that merges the return light from the detection fiber 14 and the return light from the external detection fiber 10.

The insertion portion 11 of the external detection fiber 10 is inserted into the treatment instrument insertion channel 16, and the connection portion 12 is connected to the connection portion 79 of the main body apparatus 50a. Consequently, the return light detected by the external detection fiber 10 is guided to the merging portion 15 of the main body apparatus 50a. Further, the return light from the detection fiber 14 is also guided to the merging portion 15. The merging portion 15 merges the return light from the detection fiber 14 and the return light from the external detection fiber 10 and guides the light to the demultiplexer 76. Other configurations and effects are the same as in the first embodiment.

According to the present modification, as in the first embodiment, thickening of the outer diameter of the insertion portion 2 of the endoscope 1 can be prevented, and brightness necessary for reaching the target site can be secured without application of laser light at an unnecessarily high level.

### (Second Embodiment)

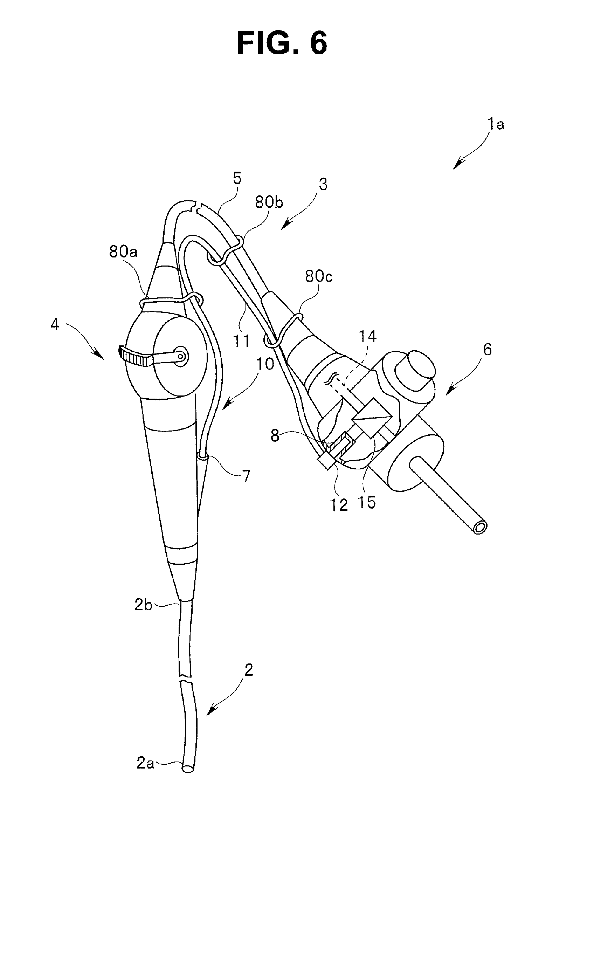

Next, a second embodiment will be described. Fig. 6 is a diagram showing a configuration of an endoscope according to the second embodiment. Note that the same components as in Fig. 1 are designated with the same reference numerals in Fig. 6, and the description will be omitted.

An endoscope 1b shown in Fig. 6 is formed by adding a plurality of hook-like locking portions 80a, 80b, and 80c to the endoscope 1 of Fig. 1. The locking portion 80a is provided to the operation portion 4, the locking portion 80b is provided to the flexible tube portion 5, and the locking portion 80c is provided to the connector 6. Note that, although the endoscope 1b includes three locking portions 80a to 80c, it is only necessary that the endoscope 1b includes at least one or more locking portions.

The locking portions 80a to 80c fix the insertion portion 11 of the external detection fiber 10 that cannot be completely inserted into the treatment instrument insertion channel 16 to arrange the insertion portion 11 along the endoscope main body portion 3.

As a result, according to the endoscope 1b of the present embodiment, the insertion portion 11 of the external detection fiber 10 that cannot be completely inserted into the treatment instrument insertion channel 16 does not distract the operator.

### (Third Embodiment)

Next, a third embodiment will be described.

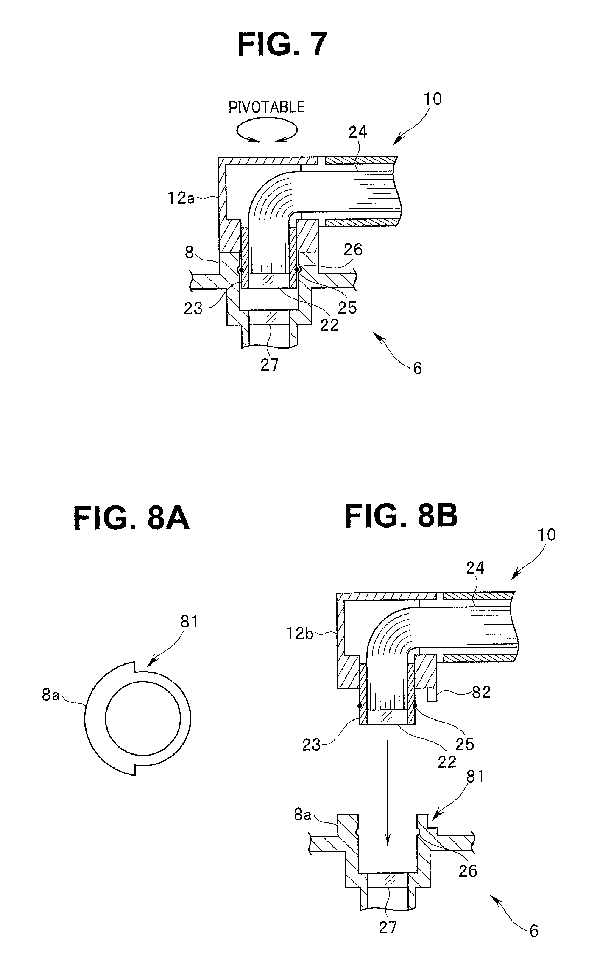

Fig. 7 is a cross-sectional view for describing a connection configuration of an external detection fiber connection portion and a connection portion of a connector. Note that the same components as in Fig. 3 are designated with the same reference numerals in Fig. 7, and the description will be omitted.

For example, in the configuration of the connection portion 12 of the external detection fiber 10 of Fig. 3, an impact on the connection portion 12 may damage the detection fiber 24 due to a bend or the like when the connection portion 12 is connected to the connection portion 8 of the connector 6.

Therefore, the external detection fiber 10 shown in Fig. 7 includes an L-shaped connection portion 12a along the endoscope main body portion 3. Such a configuration prevents damage of the detection fiber 24 due to an impact on the connection portion 12a when the connection portion 12a of the external detection fiber 10 is connected to the connection portion 8 of the connector 6.

Note that to prevent damage, such as a bend of the detection fiber 24 caused by a twist, the connection portion 12a of the external detection fiber 10 may be pivotable.

Further, to prevent an excessive twist caused by a pivotable mechanism, a mechanism for setting a rotation restriction of rotation angle within a range of 0 to 360 degrees may be provided.

Fig. 8A and Fig. 8B are diagrams for describing another connection configuration of the external detection fiber connection portion and the connection portion of the connector.

Fig. 8A is a diagram of a connection portion 8a of the connector 6 viewed from above, and the connection portion 8a includes a rotation restriction section 81 that restricts rotation of the connection portion 12a of the external detection fiber 10 within a range of up to 180 degrees.

Further, as shown in Fig. 8B, a connection portion 12b of the external detection fiber 10 includes a protrusion 82 for rotation restriction pivotably fitted with the rotation restriction section 81. When the connection portion 12b of the external detection fiber 10 is connected to the connection portion 8a of the connector 6, the protrusion 82 is fitted with the rotation restriction section 81, and the connection portion 12b rotates within a range of up to 180 degrees in a circumferential direction of the connection portion 8a.

The rotation restriction section 81 and the protrusion 82 can prevent an excessive twist of the external detection fiber 10 and can prevent a bend of the detection fiber 24.

### (Fourth Embodiment)

Next, a fourth embodiment will be described.

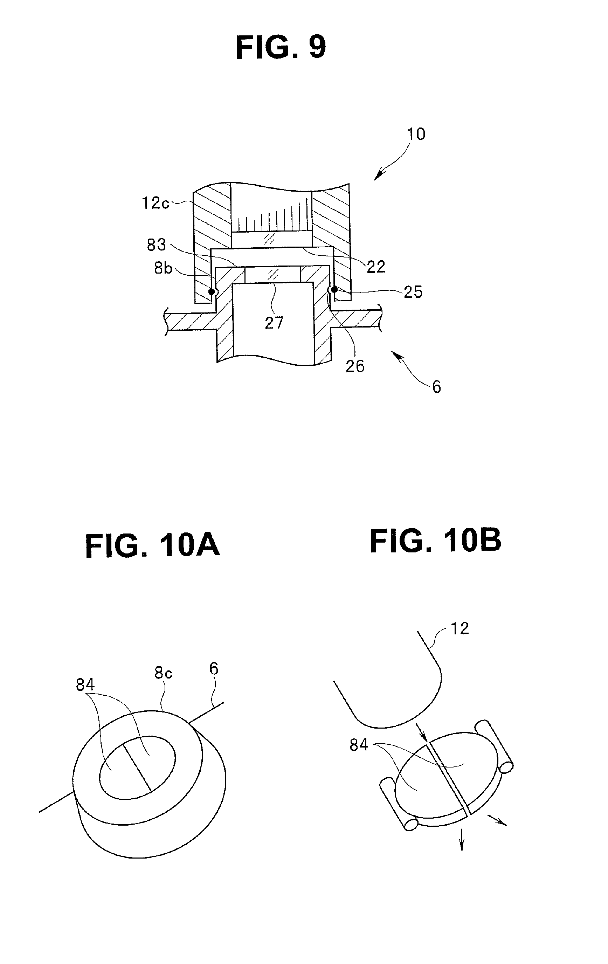

Fig. 9 is a diagram for describing a configuration of the connection portion of the connector. Note that the same components as in Fig. 3 are designated with the same reference numerals in Fig. 9, and the description will be omitted.

For example, the connection portion 8 of the connector 6 of Fig. 3 has a concave shape. Therefore, dust and the like easily enter the connection portion 8, and a stain easily adheres and remains on the cover glass 27. In this way, when a stain adheres to the cover glass 27, the return light from the external detection fiber 10 is blocked, and this causes a reduction in the brightness. That is, the brightness necessary before arrival to the target site may be insufficient.

Therefore, as shown in Fig. 9, a cover glass frame 83 that is convex to the outside of the connector 6 is provided to a distal end portion of a connection portion 8b of the connector 6, and the cover glass 27 is fixed. The connection portion 8b of the connector 6 forms a first connection portion to which the connection portion 12 (second connection portion) of the external detection fiber 10 is connected. This makes dust and the like harder to adhere to the cover glass 27.

As a result, the connection portion 8b of the connector 6 can prevent, through the cover glass frame 83, adherence and accumulation of dust and the like. Therefore, a stain does not adhere to the cover glass 27, and the brightness necessary before arrival to the target site can be secured.

Fig. 10A and Fig. 10B are diagrams for describing another configuration of the connection portion of the connector.

As shown in Fig. 10A, a shutter 84 for preventing dust and the like from entering is provided to a distal end portion of a connection portion 8c of the connector 6. Then, as shown in Fig. 10B, when the connection portion 12 connects the external detection fiber 10 to the connection portion 8c, the shutter 84 opens inside of the connection portion 8c, and the connection portion 12 is connected to the connection portion 8c of the connector 6.

As a result, the shutter 84 of the connection portion 8c of the connector 6 can prevent dust and the like from entering, just like the connection portion 8b. Therefore, a stain does not adhere to the cover glass 27, and the brightness necessary before arrival to the target site can be secured.

The present invention is not limited to the embodiments, and various changes, modifications, and the like can be made without changing the scope of the present invention.

The present application is filed on the basis of claiming the benefit of priority from Japanese Patent Application No. 2013-201899, filed September 27, 2013 in Japan, and the disclosed contents are incorporated in the present specification, claims, and drawings by reference.

## Claims

1. An endoscope comprising:
a long insertion portion that is inserted into an observed section and that includes a distal end portion and a proximal end portion;
a first light receiving portion that is provided on the distal end portion and that can receive light from an observed body;
a first light transmission portion that is inserted into the insertion portion and that transmits light received by the first light receiving portion;
a distal end side opening that opens near the first light receiving portion;
a conduit that communicates with the distal end side opening and that is inserted into the insertion portion;
a proximal end side main body portion provided on a proximal end side relative to the proximal end portion and consecutively connected with the insertion portion;
a proximal end side opening that is provided on the proximal end side main body portion and that includes an opening communicating with the conduit;
a light guiding portion that is provided on the proximal end side main body portion and that can guide light between outside and inside of the proximal end side main body portion; and
a merging portion that is provided inside of the proximal end side main body portion and that merges the light transmitted by the first light transmission portion and the light guided by the light guiding portion.

2. The endoscope according to claim 1, wherein
the proximal end side main body portion comprises: an operation portion consecutively connected with the proximal end portion of the insertion portion; a long flexible tube portion consecutively connected with a proximal end side of the operation portion; and a connector portion that is consecutively connected with a proximal end side of the flexible tube portion and that can be attached to and removed from a main body apparatus to which the light merged by the merging portion is transmitted.

3. The endoscope according to claim 1, wherein
the light guiding portion comprises a first connection portion capable of guiding light transmitted by a second light transmission portion that can be inserted into the conduit through the proximal end side opening, and the second light transmission portion can be attached to and detached from the first connection portion.

4. The endoscope according to claim 3, wherein
the second light transmission portion comprises a second light receiving portion at a distal end, and the second light receiving portion can be inserted to near the distal end side opening to receive light from the observed section.

5. The endoscope according to claim 3, wherein
a proximal end portion of the second light transmission portion includes a second connection portion to which the first connection portion is connected, and the second connection portion is provided with a cover glass at a distal end.

6. The endoscope according to claim 3, wherein
the first connection portion is provided with a cover glass at a position opposing the proximal end portion of the second light transmission portion.

7. An endoscope system comprising:
an endoscope comprising: a long insertion portion that is inserted into an observed section and that includes a distal end portion and a proximal end portion; a first light receiving portion that is provided on the distal end portion and that can receive light from an observed body; a first light transmission portion that is inserted into the insertion portion and that transmits light received by the first light receiving portion; a distal end side opening that opens near the first light receiving portion; a conduit that communicates with the distal end side opening and that is inserted into the insertion portion; a proximal end side main body portion provided on a proximal end side relative to the proximal end portion and consecutively connected with the insertion portion; a proximal end side opening that is provided on the proximal end side main body portion and that includes an opening communicating with the conduit; and a light guiding portion that is provided on the proximal end side main body portion and that can guide light between outside and inside of the proximal end side main body portion; and
a main body apparatus comprising a merging portion that merges the light transmitted by the first light transmission portion and the light guided by the light guiding portion.
